# DEMANDE DE BREVET EUROPEEN

(11) **EP 1 374 913 A1**
(43) Date de publication de la demande: **02.01.2004**
(21) Numéro de dépôt: 03291529.0
(22) Date de dépôt: 23.06.2003
(51) Int. Cl.: A61K 49/00, A61K 7/48

(54) **Procédés non thérapeutiques d'évaluation de la neuro-sensibilité cutanée, kit et utilisation d'un kit**

(30) Priorité: 25.06.2002 FR 0207869; 25.06.2002 FR 0207895
(71) Demandeur: L'OREAL, 75008 Paris (FR)
(72) Inventeur: De Lacharriere, Olivier, 75015 Paris (FR); Rubinstenn, Gilles, 75005 Paris (FR); Jourdain, Roland, 92360 Meudon la Forêt (FR)
(74) Mandataire: Allab, Myriam

(57) **Abrégé**

L'invention se rapporte à de nouveaux procédés non thérapeutiques permettent d'évaluer le niveau de neurosensibilité cutanée et/ou de l'état de réactivité cutanée neurosensorielle d'un individu par l'application sur une zone cutanée d'un agent stimulant du système nerveux périphérique, le procédé étant mis en oeuvre dans des conditions de sécurité et de confort acceptable pour l'utilisateur, quel que soit son type de peau.

## Description

L'invention se rapporte à de nouveaux procédés non thérapeutiques permettent d'évaluer le niveau de neurosensibilité cutanée et/ou de l'état de réactivité cutanée neurosensorielle d'un individu par l'application sur une zone cutanée d'un agent stimulant du système nerveux périphérique, le procédé étant mis en oeuvre dans des conditions de sécurité et de confort acceptable pour l'utilisateur, quel que soit son type de peau.

L'expérience montre que les consommateurs ne choisissent pas toujours de manière optimale les produits cosmétiques ou de soins de la peau, parce qu'ils ne connaissent pas précisément leur type ou nature de peau et ses besoins spécifiques. Ceci est particulièrement vrai pour les peaux dites sensibles pour lesquelles la capacité d'autoappréciation est très limitée.

Il existe par conséquent un besoin pour permettre la détermination du niveau de neurosensibilité cutanée, la meilleure connaissance du niveau de neurosensibilité cutanée pouvant guider les consommateurs dans le choix de produits qui leur sont adaptés ou le formulateur dans la préparation de produits ciblés, voire personnalisés.

Il est connu que certaines personnes ont un niveau de neurosensibilité cutanée très bas et réagissent à des substances irritantes ou à des facteurs environnementaux (pollution, soleil, eau de mer, froid sec) de manière beaucoup plus intense que d'autres personnes ayant un niveau de neurosensibilité cutanée plus élevé: c'est notamment le cas des personnes dites à peaux sensibles ou réactives.

La neurosensibilité cutanée, en particulier la peau sensible ou réactive, n'est pas un état pathologique mais correspond à un paramètre qui traduit un état constitutif de la peau. La neurosensibilité cutanée est un état constitutif de la peau au même titre que la peau grasse ou la sèche par exemple.

La peau sensible se définit comme une peau hyperréactive à différents facteurs agissant par des mécanismes non immunologiques. On doit concevoir cette hyperréactivité comme un abaissement du seuil de tolérance de la peau à des stimuli habituellement bien tolérés. Ces stimuli sont habituellement bien tolérés. Ces stimuli sont d'origine externe, de nature physique ou chimique. Dans le langage des consommateurs, les termes « peau sensible » et « peau réactive » sont quasiment équivalents, et on admet qu'ils sont synonymes; on peut ainsi parler indifféremment de peau sensible ou de peau réactive.

Plusieurs décennies après les premières publications concernant la peau sensible, le point de consensus sur lequel la plupart des auteurs s'accorde est l'authenticité de ce syndrome. Il est intéressant de remarquer que les ouvrages de dermatologie ne se réfèrent jamais à la « peau sensible » ni à aucun syndrome clinique équivalent, laissant clairement apparaître le caractère cosmétique de cette entité. Ceci est peut-être à l'origine de la méconnaissance de ce syndrome par une grande partie du monde dermatologique.

Des études épidémiologiques effectuées en Europe et aux Etats-Unis retrouvent des fréquences équivalentes. Environ la moitié des femmes et un tiers des hommes déclarent avoir la peau sensible; 10% des femmes et 6% des hommes déclarent avoir la peau très sensible. Avec l'augmentation en âge, la fréquence de la peau sensible tend à diminuer.
La plus grande fréquence des peaux sensibles chez les femmes explique que la plupart des études concernant les peaux sensibles leur ait été consacrée.

Les individus à peau sensible se plaignent en premier lieu de signes d'inconfort cutanés. Cet « inconfort cutané » se traduit par des signes neuro-sensoriels qui sont des sensations d'échauffements, de picotements, de fourmillements ou de démangeaisons. Dans la très grande majorité des cas cette symptomatologie est strictement localisée auvisage. Toutefois environ 25 % des hommes et des femmes se plaignent d'avoir un scalp sensible. Chez certains individus, l'hyper-réactivité sensitive peut atteindre d'autres zones du corps, mais l'atteinte du visage y est toujours associée et reste prépondérante.

La survenue de cette symptomatologie est déclenchée par plusieurs types de facteurs. Il peut soit s'agir de facteurs environnementaux (variations de températures, chaleur, froid, vent, soleil, pollution atmosphérique....), soit de l'application de certains produits topiques, d'eau « dure » (à forte concentration en calcium) ou encore de facteurs internes (facteurs émotionnels, cycles menstruels, facteurs alimentaires...).

La parenté étymologique entre les termes "sensible" et "sensibilisation" est certainement à prendre en compte dans la confusion qui règne encore entre "peaux allergiques" et "peaux sensibles". Dès 1962, H. Thiers (Peau sensible.ln: Thiers Héd. Les cosmétiques, 2^{e} éd, Paris : Masson, 1986 : 266-68) a bien insisté sur le fait que les peaux sensibles n'étaient pas liées à une manifestation immunologique.

La demanderesse a pu montrer que l'intensité des manifestations et les facteurs de réactivité varient d'un sujet à l'autre et permettre de décrire plusieurs formes cliniques.

### Les peaux très sensibles

Elles concernent environ 10% des femmes et 6% des hommes. Ces peaux très sensibles se traduisent par une très grande réactivité de la peau du visage aussi bien à des produits topiques qu'à des facteurs environnementaux (y compris la pollution atmosphérique) ainsi qu'à des facteurs internes tel que le stress ou les états de fatigue. Parmi les peaux très sensibles on retrouve aussi bien des peaux sèches que des peaux grasses.
Ces peaux très sensibles peuvent présenter des états de crise qui peuvent s'étendre sur des périodes de plusieurs jours, voir de plusieurs semaines. Au cours de ces périodes de crise, l'hyper-réactivité cutanée est extrême, la peau devenant intolérante à toute application épicutanée, y compris les produits habituellement bien tolérés en période normale. Cet état d'intolérance cutanée extrême, ou « status cosmeticus » se traduit par l'apparition de l'ensemble des signes d'inconfort associés à un érythème dés qu'un produit est appliqué sur la peau. Ces états d'intolérance cutanée sont souvent très déconcertants pour les patients et les dermatologues.

### Les peaux sensibles environnementales

Environ 15 à 20 % des femmes présentent une peau sensible réagissant principalement aux facteurs environnementaux tels que la chaleur ou les variations de températures brusques. Les femmes présentant ce type de peaux sensibles ont souvent un aspect porcelainé de la peau et se plaignent parfois d'intolérance au soleil, mais bien que leur carnation soit plutôt claire, il semble plutôt s'agir d'une sensibilité à la chaleur plutôt que d'une sensibilité aux ultraviolets. C'est parmi ces peaux sensibles que l'on retrouve plus fréquemment des peaux sèches et des peaux rougissant facilement.

### Les peaux sensibles cosmétiques

Environ 25% des femmes présentent une peau sensible réagissant principalement à des applications épicutanées. Le facteur déclenchant est ici principalement l'application de produits contenant un actif mal toléré chez ces individus. Il est important de préciser que cette intolérance, source de l'inconfort cutané parfois (mais pas toujours) associé à une rougeur, ne rentre pas dans le cadre d'un mécanisme allergique. L'apparition de l'état d'inconfort cutané suit immédiatement ou dans les minutes qui suivent l'application du produit, et ceci dés la première application. Il faut souligner que cet état est également bien différent des états d'intolérance extrême du « status cosmeticus », l'intolérance étant ici limitée à un ou quelques produits facilement identifiables.

On connaît déjà des méthodes d'évaluation de la réactivité cutanée qui utilise des agents irritants.

On peut citer le test à l'acide lactique, ou *Stinging test,* qui est décrit par Frosch P.J., Kligman A.M., dans le document J. Soc. Cosmet. Chem., 1977, 28:197-209.
Ce test, réalisé au niveau du sillon naso-génien consiste, à quantifier les picotements (stinging) apparaissant après l'application d'une solution d'acide lactique à 10%. Le protocole le plus couramment utilisé actuellement consiste à évaluer les picotements toutes les minutes sur une échelle de 0 à 3, pendant 5 minutes en se référant au côté hétérolatéral qui sert de témoin, sur lequel la solution véhicule est appliquée. Le score retenu est le suivant (Somme des scores du côté acide lactique - Somme des scores du côté témoin). Toutefois, son caractère diagnostique ne fait pas l'unanimité car tous les « stingers » ne sont pas des individus à peau sensible (voir le document Contact Dermatitis, 1998, 38:311-315).

Par ailleurs, le document EP 0 680 749 A2 décrit un test à la capsaïcine pour caractériser les personnes à peau sensibles grâce à la réponse neuro-sensorielle induite par l'application cutanée de capsaïcine. Les individus à peau sensible montrent en effet des niveaux d'inconfort cutané plus important que ceux à peau non sensible après application d'une crème à la capsaïcine. Ce test consiste à appliquer sur environ 4 cm² de peau, 0,05 ml d'une crème contenant 7.5 10⁻²% de capsaïcine et à noter l'apparition de signes subjectifs provoqués par cette application, tels que picotements, brûlures et démangeaisons
Contrairement au test à l'acide lactique, ce test a un caractère prédictif pour le diagnostic de la peau sensible. Ce test constitue bien une amélioration pour le diagnostic de la peau sensible mais présente encore des inconvénients. En effet, le test est long dans sa mise en oeuvre (environ 30 minutes) et ne permet pas d'être utilisé pratiquement et simplement par les consommateurs pour auto-diagnostiquer leur peau sensible. En outre, le test décrit dans EP 0 680 749 A2 nécessite d'utiliser une crème dont la quantité à appliquer doit être quantifiée à l'aide d'une seringue de 1ml et nécessite l'application de la crème sur surface bine précise de la peau. En fait, la réalisation pratique de ce test nécessite la présence d'un expérimentateur réalisant le test sur un individu.
Le protocole du test de EP 0 680 749 A2 est donc difficile à mettre en oeuvre par une personne non habituée, il est de ce fait particulièrement inadapté à une utilisation par un consommateur en auto-diagnostic.

Il est également connu une méthode qui permet de corréler le profil de réponse concernant la self-perception de sensations et les seuils de détection de la capsaïcine (B.G. Green *et al*., *J. Soc. Cosmet. Chem.,* 43, 131-147, 1992). Dans cette méthode, les sujets testés situent sur une échelle les sensations cutanées imaginées (pas de sensation, à peine détectable, faible, modérée, forte, très forte, la plus forte imaginable) qu'ils ressentiraient suite à des stimuli cutanés hypothétiques fournis dans un questionnaire. L'étude se poursuit sur les mêmes sujets par des applications itératives de quantités croissantes de capsaïcine jusqu'à ce que le sujet testé ressente des sensations modérées ou fortes.

On peut encore citer le document Chemical Sensés, 13(3), 367-384, 1988, qui étudie la nature et l'intensité des sensations induites par l'application topique de solutions hydroalcooliques de capsaïcine sur l'avant bras. Le but de cette étude est de déterminer un seuil global de sensibilité à la capsaïcine en appliquant sur les sujets des concentrations croissantes de capsaïcine (une concentration unique est testée par jour). La méthode décrite comprend nécessairement l'application d'une première concentration en capsaïcine de 0.05%.

Cependant, la mise en oeuvre des méthodes de l'art antérieur n'est pas appropriée chez les personnes ayant un niveau de neurosensibilité cutané bas, les dites personnes constituant le coeur de cible des produits "peaux sensibles". En effet, dans ces méthodes, les concentrations utilisées en agent irritant (capsaïcine) sont telles qu'elles provoquent d'emblée chez ces personnes des sensations douloureuses et/ou des réactions d'irritation souvent intolérables. Ceci est particulièrement vrai chez les personnes qui ignorent qu'elles présentent de manière constitutive un seuil de neurosensibilité cutanée particulièrement bas.

L'invention vise à accroître encore la diversité des procédés et des dispositifs à la disposition du public et des professionnels pour évaluer le niveau de neurosensibilité cutanée d'un individu, en particulier l'état de réactivité cutané ou de peau sensible, à rendre les dits procédés suffisamment simples et rapides dans leur mis en oeuvre et à permettre leur utilisation à grande échelle.

De manière surprenante et inattendue, la Demanderesse a maintenant découvert de nouveaux procédés non thérapeutiques d'évaluation de la neurosensibilité cutanée, en particulier l'état de réactivité cutané ou de peau sensible, cette évaluation étant réalisée :
(i) soit par l'application topique cutanée unique d'un véhicule comprenant une concentration d'un agent stimulant du système nerveux périphérique, et déduire une information quant à la réactivité ou la sensibilité cutanée d'un individu en fonction de l'intensité des sensations dysesthésiques susceptibles d'être ressenties par ledit individu;
(ii) soit par des applications successives d'un véhicule comprenant des concentrations croissantes d'agent stimulant du système nerveux périphérique, jusqu'à ce qu'une sensation, aussi minime soit-elle, soit perçue par le sujet ou bien qu'une concentration maximale n'ayant pas induit de sensation ait été appliquée, le test prenant fin à ce stade, bien avant d'être dans le registre de la douleur, Ainsi, les procédés selon l'invention sont compatibles avec un outil de diagnostic cosmétique car réalisés dans des conditions de confort acceptable par tout type d'individu, sans qu'une douleur soit ressentie au cour de leur mise en oeuvre et dans des conditions de sécurité totale et sans générer d'effets secondaires. En outre, les procédés de l'invention suivant (i) sont plus faciles à mettre en oeuvre, plus discriminantes et plus précises par rapport aux tests connus de l'art antérieur et permettent ainsi d'améliorer l'identification des personnes présentant une peau sensible ou réactive. Les méthodes suivant la présente invention sont particulièrement adaptées à une utilisation par des consommateurs en auto-diagnostic.

L'invention a notamment pour objet un procédé non thérapeutique d'évaluation du niveau de neurosensibilité cutanée d'un individu consistant à :
1) appliquer sur une zone cutanée dudit individu une composition contenant un véhicule physiologiquement acceptable et un agent stimulant du système nerveux périphérique, la concentration dudit agent étant comprise entre 1.10⁶ et 5.10⁻⁴% en poids par rapport au poids total de la composition;
2) noter si l'individu détecte ou ressent une sensation dysesthésique et en déduire une information quant à la neurosensibilité cutanée de l'individu, avantageusement de la réactivité ou la sensibilité cutanée.

Dans un premier mode de réalisation préféré, l'invention a pour objet un procédé non thérapeutique d'identification des personnes à peaux sensibles consistant à:
1) appliquer sur une zone cutanée d'un individu, avantageusement le pli du bras, le lobe de l'oreille ou la face postérieure du pavillon de l'oreille ou le visage, en particulier l'aile du nez ou le sillon naso-génien, une solution aqueuse ou hydroalcoolique, avantageusement hydroéthanolique, contenant de 1 à 50% d'éthanol dans l'eau, avantageusement de 5% à 20%, de préférence 10% d'éthanol, d'un agent choisi parmi les capsaicinoides ou l'huile de moutarde à une concentration comprise entre 1.10⁻⁶ et 5.10⁻⁴%, avantageusement entre 5.10⁻⁵ et 5.10⁻⁴%; de préférence la concentration est de 1.10⁻⁴% et ;
2) déduire une information quant à la réactivité ou la sensibilité cutanée d'un individu en fonction de l'intensité des sensations dysesthésiques susceptibles d'être ressenties par ledit individu.

Avantageusement, suivant le premier mode de réalisation de l'invention, l'étape 1) comprend entre 1 et 3 application(s) de la solution, de préférence 3 applications.

L'application de la solution hydroalcoolique de capsaïcine se fait par un système mono-applicateur, notamment à l'aide de coton-tiges ou d'un morceau de coton (type coton à démaquiller circulaire) plié en 4.

Les pourcentages qui sont donnés correspondent au pourcentage en poids par rapport au poids total de la composition.

Avantageusement, l'étape 1) du procédé suivant le premier mode de réalisation est précédé d'une étape 0) consistant à appliquer sur une zone cutanée d'un individu, avantageusement le pli du bras, le lobe de l'oreille ou derrière le pavillon de l'oreille ou le visage, en particulier sur l'aile du nez ou le sillon naso-génien, une solution d'acide lactique à une concentration comprise entre 2 et 10%, avantageusement à 10%.
Ainsi, un procédé non thérapeutique d'identification des personnes à peaux sensibles préféré consiste à:
0) appliquer sur une zone cutanée d'un individu, avantageusement le pli du bras, le lobe de l'oreille ou derrière le pavillon de l'oreille ou le visage, en particulier sur l'aile du nez ou le sillon naso-génien, une solution d'acide lactique à une concentration comprise entre 2 et 10%, avantageusement à 10% puis;
1) appliquer sur une zone cutanée du même individu, avantageusement le pli du bras, derrière le lobe ou le visage, en particulier sur l'aile du nez ou le sillon naso-génien, une solution aqueuse ou hydroalcoolique, avantageusement hydroéthanolique, contenant de 1 à 50% d'éthanol dans l'eau, avantageusement de 5% à 20%, de préférence 10% d'éthanol, d'un agent choisi parmi les capsaicinoides ou l'huile de moutarde à une concentration comprise entre 1.10⁻⁶ et 5.10⁻⁴%, avantageusement entre 5.10⁻⁵ et 5.10⁻⁴%; de préférence la concentration est de 1.10⁴%;
2) déduire une information quant à la réactivité ou la sensibilité cutanée d'un individu en fonction de l'intensité des sensations dysesthésiques susceptibles d'être ressenties par ledit individu.

Avantageusement, l'étape 0) comprend entre 1 et 10 application(s) de solution d'acide lactique, avantageusement 10 applications.

Avantageusement, l'étape 1) comprend entre 1 et 3 application(s) de l'agent, avantageusement 3 applications.

Les deux procédés précités présentent l'avantage de pouvoir être facilement et rapidement mise en oeuvre pour un coût industriellement acceptable tout en permettant d'obtenir des informations suffisamment précises et rapides pour permettre un diagnostic aisé de l'existence d'un état de peau sensible ou réactive.

En outre, ces deux procédés précités permettent tous les deux de diagnostiquer un état de réactivité cutanée ou de peau sensible d'un individu et ne diffèrent l'un de l'autre que par l'utilisation d'une solution de capsaïcine seule ou d'une solution d'acide lactique puis d'une solution de capsaïcine: le premier procédé est plus simple d'utilisation, le second procédé est plus discriminant et précis.

Dans un second mode de réalisation préféré, l'invention a pour objet un procédé non thérapeutique d'évaluation du niveau de neurosensibilité cutanée d'un individu consistant à :
1) appliquer sur une zone cutanée dudit individu une première composition contenant un véhicule physiologiquement acceptable et un agent stimulant du système nerveux périphérique, la concentration dudit agent étant comprise entre 1.10⁻⁶ et 1.10⁻⁴% en poids;
2a) noter si l'individu détecte une sensation dysesthésique,
2b) si aucune sensation n'est détectée par l'individu, recommencer les étapes 1) et 2a) avec une composition contenant une concentration plus élevée du même agent jusqu'à ce que l'individu détecte une sensation dysesthésique ou jusqu'à ce qu'à ce qu'une composition contenant une valeur maximale de concentration dudit agent soit appliquée;
2c) déduire, de la dernière concentration appliquée, une information quant à la neurosensibilité cutanée de l'individu.

Avantageusement selon ce second mode de réalisation de l'invention, la concentration de l'agent dans la composition qui est appliquée à l'étape 2b) est telle que son application n'est pas susceptible d'engendrer des sensations dysesthésiques douloureuses chez l'individu. Préférentiellement, la concentration de l'agent dans la composition qui est appliquée à l'étape 2b) augmente d'un facteur compris entre 1,5 à 10, de préférence d'un facteur compris entre 2 et 5, avantageusement elle augmente d'un facteur de racine carrée de 10.

Dans le procédé suivant le second mode de réalisation l'invention, la valeur maximale de concentration en agent stimulant du système nerveux périphérique qui est susceptible d'être appliqué est de 1.10⁻²% en poids.

Par véhicule physiologiquement acceptable on entend un véhicule compatible avec la peau, les muqueuses, les ongles, les cheveux. En outre, ce véhicule est approprié à l'application de l'agent stimulant du système nerveux périphérique et permet une bonne biodisponibilité dudit agent après application topique sur la peau, ce véhicule n'étant pas par lui-même un stimulant du système nerveux périphérique.

Les véhicules selon l'invention correspondent à des formes galéniques bien connus de l'homme du métier. Ces véhicules peuvent se présenter sous toutes les formes galéniques normalement utilisées pour une application topique: il peut s'agir notamment de solutions aqueuses, hydroalcooliques ou huileuses, de dispersions du type lotion ou sérum, de gels anhydres ou lipophiles, d'émulsions de consistance liquide ou semi-liquide du type lait, obtenues par dispersion d'une phase grasse dans une phase aqueuse (H/E) ou inversement (E/H), ou de suspensions ou d'émulsions de consistance molle, semi-solides ou solides, ou encore de microémulsions, de microcapsules, de microparticules ou des dispersions vésiculaires de type ionique et/ou non ionique, avantageusement, de solutions, de préférence hydroalcooliques avec une teneur en alcool inférieure à 50%. Ces compositions sont préparées selon les méthodes usuelles.

Par agent stimulant du système nerveux périphérique, on entend un agent qui induit une réponse sensorielle liée à la mise en jeu de nerfs sensitifs cutanés, dont les terminaisons affleurent au niveau du stratum corneum
L'agent stimulant du système nerveux périphérique est une substance capable d'induire une sensation dysesthésique lorsqu'il est appliqué topiquement sur la peau. En outre, ledit agent est capable d'induire une libération de substance P et/ou de CGRP (peptide dérivé du gène de la calcitonine : Calcitonin Gene Related Peptide en langue anglosaxonne) lorsqu'il est appliqué topiquement sur la peau. La libération de ces peptides peut être obtenue par réaction avec des anticorps monoclonaux dans les cas de la substance P (selon une méthode décrite par Cuello et al. dans Proc. Natl. Acad. Sci. USA 1979 ; 76 : 3532-6) ou par la technique dite de Radioimmunoassay pour la substance P et le CGRP (méthode décrite par Wallendren et al. dans Acta Derm Venereol 1987; 67: 185-92), cette deuxième technique étant particulièrement adaptée à la peau.

Un agent stimulant du système nerveux périphérique est avantageusement choisi parmi les capsaicinoides naturels ou synthétiques, de préférence la capsaïcine, l'homocapsaïcine, homodihydrocapsaïcine, la nordihydrocapsaïcine, la dihydrocapsaïcine, avantageusement la capsaïcine; l'acide lactique, l'acide glycolique, l'éthanol à une concentration supérieure à 50%, l'huile de moutarde.

Avantageusement, la concentration de la première composition appliquée est comprise entre 1.10⁻⁶ et 1.10⁻⁴%, avantageusement 3.10⁻⁶ et 6.10⁻⁵% ; préférentiellement 3.16 10⁻⁵%.

Avantageusement, la zone cutanée selon l'invention est le pli du bras, le lobe de l'oreille ou le visage, en particulier l'aile du nez, le sillon naso-génien ou l'angle du maxillaire inférieur.

Lorsque l'agent stimulant du système nerveux périphérique est la capsaïcine, un véhicule préféré est une solution hydroalcoolique, avantageusement hydroéthanolique, comprenant de 1% à 50% d'éthanol dans l'eau, avantageusement de 5% à 20%, préférentiellement 10%. Ces solutions hydroéthanoliques de capsaïcine sont particulièrement stables dans le temps pour des concentrations en capsaïcine comprises entre 1.10⁻²% et 1.10⁻⁶%.

Au sens de la présente invention on entend par sensation dysesthésique la plus petite sensation non douloureuse ressentie dans une zone traitée par l'agent stimulant évoquant des picotements, des fourmillements, des démangeaisons ou prurits, des échauffements, des tiraillements, et/ou toute autre gêne ou inconfort telle qu'une sensation d'occlusion et/ou de vibrations. Une telle sensation dysesthésique est susceptible d'être détectée par un individu suite à l'application de composition comprenant l'agent stimulant du système nerveux périphérique à une concentration comprise entre 1.10⁻⁶ et 1.10⁻⁴% en poids.

La concentration de la première composition (appelée concentration d'attaque) qui contient l'agent stimulant du système nerveux périphérique et qui est utilisée dans la méthode suivant l'invention a été déterminée au préalable pour n'induire que des sensations dysethésiques chez les personnes ayant le degré de neurosensibilité cutanée le plus élevé.
Cette première concentration (concentration d'attaque optimale) a été déterminée en utilisant comme véhicule une solution hydroéthanolique à 10% d'éthanol (Exemple 1).

La capsaïcine est la substance de référence qui est utilisée comme agent stimulant du système nerveux périphérique. La capsaïcine est la principale substance piquante des piments. Il est possible d'estimer le pouvoir piquant d'une substance à l'aide du test de Scoville (http://pubs.acs.org/hotartcl/tcaw/00/may/dong.html). Ce test établit que la capsaïcine pure présente le Scoville le plus élevé (16 millions de Scoville) parmi toutes les substances piquantes connues ayant été testées. Dès lors, on comprend que d'autres agents stimulant du système nerveux périphérique, puissent être utilisés à la même concentration d'attaque que la capsaïcine (1.10⁻⁶ et 1.10⁻⁴%, avantageusement entre 3.10⁻⁶ et 6.10⁻⁵ %; préférentiellement 3.16 10⁻⁵%) sans induire d'emblée des sensations de douleur.

Les pourcentages qui sont donnés correspondent au pourcentage en poids par rapport au poids total de la composition.

Suivant l'invention, la dernière concentration testée de l'étape 2b) peut correspondre à la concentration maximale ou à la concentration ayant engendrée la dite sensation dysesthésique.

Dans un autre mode de réalisation, le procédé suivant l'invention comprend avant l'étape 1) l'application préalable sur une zone cutanée d'une composition comprenant le véhicule sans agent stimulant.
Dans un autre mode de réalisation de l'invention, le procédé comprend avant l'étape 1), l'application préalable sur une zone cutanée et sur sa zone contralatéral d'une composition comprenant le véhicule sans agent stimulant.

Dans un mode d'exécution préféré de l'invention le procédé non thérapeutique comprend les étapes successives suivantes:
a1) appliquer sur une zone cutanée, une composition contenant le véhicule;
b1) noter si le sujet a ressenti une sensation dysesthésique sur la zone cutanée ayant reçue le véhicule ;
c1) si oui, arrêter le test ; Si non, appliquer sur une zone cutanée, de préférence sur la même zone ayant reçue précédemment le véhicule, le même véhicule contenant ledit agent stimulant à une concentration comprise entre 1.10⁻⁶ et 1.10⁻⁴% ;
d1) noter si le sujet a ressenti une sensation dysesthésique, sur la zone cutanée ayant reçue la composition contenant l'agent stimulant;
e1) si oui, noter la concentration en agent stimulant et arrêter le test, si non, incrémenter la concentration en agent stimulant d'un facteur compris entre 1,5 et 10, et répéter n fois les étapes c1) à e1), avec n compris entre 1 et 10.

Avantageusement, après l'étape a1) et avant l'étape b1) et/ou après l'étape c1) et avant l'étape d1), attendre 30 à 360 secondes, préférentiellement 120 à 200 secondes, particulièrement 180 secondes.

Dans un autre mode d'exécution préféré de l'invention le procédé non thérapeutique comprend les étapes successives suivantes:
a2) appliquer sur une zone cutanée et sur sa zone contralatérale, une composition contenant le véhicule;
b2) noter si le sujet a ressenti une sensation dysesthésique, sur l'une au moins des zones du visage ayant reçue le véhicule;
c2) si oui arrêter le test; si non, appliquer sur une zone cutanée, le même véhicule contenant ledit agent stimulant à une concentration d'attaque comprise entre 1.10⁻⁶ et 1.10⁻⁴ %; et appliquer le même véhicule sur la zone contralatérale;
d2) noter si le sujet a ressenti une sensation dysesthésique discriminante, sur la zone cutanée ayant reçue le véhicule contenant l'agent stimulant par rapport à la zone cutanée contralatérale ;
e2) si oui, noter la concentration en agent stimulant et arrêter le test, si non, incrémenter la concentration en agent stimulant d'un facteur compris entre 1,5 à 10, et répéter n fois les étapes 3) à 5), avec n compris entre 1 à 10.

Avantageusement, après l'étape a2) et avant l'étape b2) et/ou après l'étape c2) et avant l'étape d2), attendre 30 à 360 secondes, préférentiellement 120 à 200 secondes, particulièrement 180 secondes.

Les méthodes suivant le second mode de réalisation de l'invention présentent l'avantage de pouvoir être facilement et rapidement mises en oeuvre pour un coût industriellement acceptable tout en permettant d'obtenir des informations suffisamment précises et rapides pour permettre un diagnostic aisé du degré de neurosensibilité cutanée. Les méthodes suivant le second mode de réalisation de l'invention présentent en outre l'avantage de montrer une bonne répétabilité au sens clinique du terme.

L'invention a encore pour objet des procédés de traitement cosmétique de la peau d'un individu qui comprend l'un quelconque des procédés précédemment décrits, et une étape consistant à traiter cosmétiquement la peau avec un produit cosmétique en fonction de la neurosensibilité cutanée qui a été évaluée.

Pour comprendre ce que l'on entend par cosmétique au sens de la présente invention, on pourra se référer à la Directive cosmétique 76/768/CEE.
Par "traitement cosmétique" on entend tout traitement non thérapeutique au moyen d'un produit cosmétique tel que défini dans la Directive ci-dessus.

L'invention a encore pour objet des procédés pour déterminer l'efficacité d'un traitement cosmétique apte à agir sur la neurosensibilité cutanée d'un individu, qui comprennent l'un quelconque des procédés précédemment décrits, à traiter cosmétiquement la peau avec un produit cosmétique en fonction de la neurosensibilité cutanée qui a été évaluée, renouveler sur la peau traitée l'un quelconque des procédés précédemment décrits, puis déduire de la comparaison des résultats avant et après traitement une indication relative à l'efficacité du traitement cosmétique.

L'invention a encore pour objet un kit comprenant:
- une pluralité de récipients qui contiennent chacun des concentrations croissantes d'agent stimulant du système nerveux périphérique en association avec un véhicule,
- au moins un récipient qui contient le véhicule seul et,
- des systèmes mono-applicateurs, préférentiellement des cotons-tige,
caractérisé en ce qu'au moins un récipient contient une concentration en agent stimulant du système nerveux périphérique comprise entre 1.10⁻⁶ et 1.10⁻⁴ % en poids, avantageusement entre 3.10⁻⁶ et 6.10⁻⁵% ; préférentiellement 3.16 10⁻⁵% en poids par rapport au poids total de la composition.

Dans le kit, l'agent stimulant du système nerveux périphérique et le véhicule ont les mêmes définitions que celles décrites précédemment.

Par récipient au sens de la présente invention, on entend toute sorte de contenant quelle qu'en soit la forme. A titre d'exemple non limitatif il peut s'agir d'un pot, d'une bouteille, d'un flacon, d'un sachet, d'un tube, d'une capsule.

Avantageusement, le kit comprend
- un ou plusieurs jeux de 3 à 6 récipients contenant respectivement des concentrations en agent stimulant, choisies parmi 3,16.10⁻⁵% (C1), 1.10⁻⁴% (C2), 3,16.10⁻⁴% (C3), 1.10⁻³% (C4), 3,16.10⁻³% (C5) et 1.10⁻²% (C6), dans un véhicule,
- un récipient contenant le véhicule seul et,
- des cotons tiges,
   étant entendu que le kit comprend au moins un récipient à une concentration d'attaque comprise entre 1.10⁻⁶ et 1.10⁻⁴ %, avantageusement entre 3.10⁻⁶ et 6.10⁻⁵ %; préférentiellement 3.16 10⁻⁵ %.

Dans une variante, le kit comprend :
- un récipient contenant une solution mère d'agent stimulant à 1.10²% dans un véhicule,
- au moins un récipient contenant le véhicule seul.
- au moins un récipient vide;
- une notice contenant un mode d'emploi pour la préparation et la flaconnage des solutions choisies C1-6 telles que définies précédemment; étant entendu que le mode d'emploi comprend la réalisation d'au moins un récipient avec une concentration d'attaque comprise entre 1.10⁻⁶ et 1.10⁻⁴%, avantageusement entre 3.10⁻⁶ et 6.10⁻⁵%; préférentiellement 3.16 10⁻⁵%.

Avantageusement, le kit comprend en outre une crème émolliente pour traiter la zone d'application.

Avantageusement, le kit comprend en outre un mode d'emploi du kit reprenant les grandes lignes d'un des protocoles tels que décrits précédemment, des conseils et conclusions à donner à la personne sur le choix d'un produit cosmétique et/ou des recommandations sur ses habitudes et gestes cosmétiques, en fonction de sa neurosensibilité cutanée ainsi évaluée.

L'invention a encore pour objet l'utilisation du kit dans l'un quelconque des procédés d'évaluation précédemment décrits.

Les exemples qui suivent illustrent l'invention sans en limiter en aucune façon la portée.

### EXEMPLE 1: Détermination de la concentration d'attaque et du saut de concentration optimum avec une solution hydroalcoolique de capsaïcine

Le but de cette étude était de fixer une concentration initiale de début de test optimale, n'induisant pas de douleur et de fixer un saut de concentration suffisamment grand pour ne pas trop augmenter le nombre d'étapes successives et suffisamment petit pour qu'à la concentration n+1, le test ne soit pas douloureux en cas de non perception de sensations dysesthésiques à l'étape n.

### Solutions de capsaïcine

La capsaïcine a été testée sous forme de solutions hydroalcooliques (éthanol 10%, eau 90%),

### Design expérimental

- Etude randomisée, en simple aveugle, contrôlée
- 13 sujets sains, des 2 sexes
- 4 solutions de capsaïcine testées : 3,3.10⁻⁵% (C1), 1.10⁻⁴% (C2), 3,3.10⁻⁴% (C3), 1.10⁻³% (C4)

1^{ère} étape : Application simultanée sur les sillons naso-géniens de chaque côté de visage, du véhicule à l'aide de coton-tiges imprégnés pour habituer le volontaire aux sensations induites par l'application du véhicule. Attente de 3 minutes.

2^{ème} étape: Application du véhicule d'un côté et de la solution de capsaïcine à la concentration C1 de l'autre. Au bout de 3 minutes, demander si le sujet a senti une différence entre les 2 côtés. Si le sujet décrit une sensation dysesthésique du côté ayant reçu la capsaïcine, arrêter le test et attribuer C1 comme seuil de détection. Si non, aller immédiatement à l'étape 3.

3^{ème} étape : Application de la solution C2 du côté ayant reçu le véhicule à l'étape 1 et du véhicule de l'autre côté. Au bout de 3 minutes, demander si le sujet a senti une différence entre les 2 côtés. Si le sujet décrit une sensation particulière du côté ayant reçu la capsaïcine, arrêter le test et attribuer C2 comme seuil de détection. Si non, aller immédiatement à l'étape 4.

4^{ème} étape: Application de la solution C3 du côté ayant reçu le véhicule à l'étape 2 et du véhicule de l'autre côté. Au bout de 3 minutes, demander si le sujet a senti une différence entre les 2 côtés. Si le sujet décrit une sensation particulière du côté ayant reçu la capsaïcine, arrêter le test et attribuer C3 comme seuil de détection. Si non, aller immédiatement à l'étape 5.

5^{ème} étape: Application de la solution C4 du côté ayant reçu le véhicule à l'étape 3 et du véhicule de l'autre côté. Au bout de 3 minutes, demander si le sujet a senti une différence entre les 2 côtés. Si le sujet décrit une sensation particulière du côté ayant reçu la capsaïcine, attribuer C4 comme seuil de détection. Si non, lui attribuer comme seuil de détection : concentration > C4.

Durée du test: Explications initiales + 5 étapes de 3 à 4 minutes chacune avec une durée maximale du test de 20 à 25 minutes pour les sujets allant jusqu'à la dernière étape.

### Résultats

Les informations recueillies auprès des sujets concernant leur appréciation du test révèlent que le test a été absolument non douloureux pour l'ensemble des 13 sujets et que le saut de concentration est suffisamment faible pour éviter une sensation douloureuse avec la concentration Cn+1 quand un sujet n'a pas perçu de sensation dysesthésique au préalable Cn.

### Conclusions

Les résultats ce test permettent ainsi de déterminer une concentration d'attaque optimale de 10.10⁻⁵% et un saut adéquat entre concentration de 10.

### EXEMPLE 2 : Exemple de réalisation d'un kit visant à détecter le niveau de peau sensible, le kit étant destiné à un utilisateur professionnel avancé.

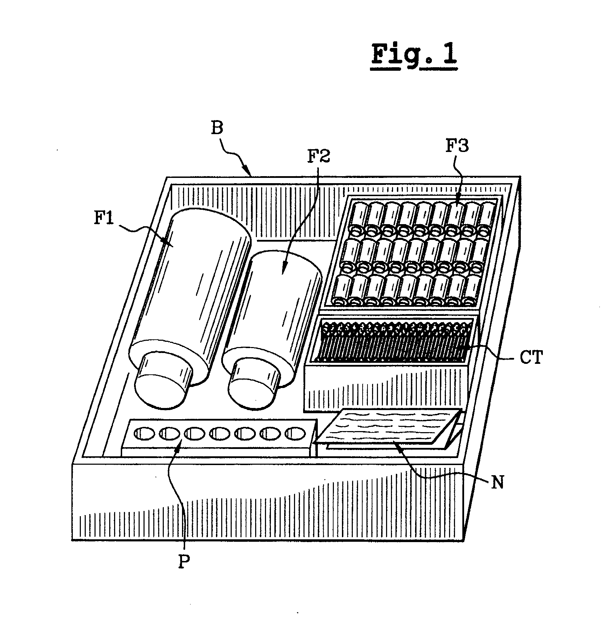

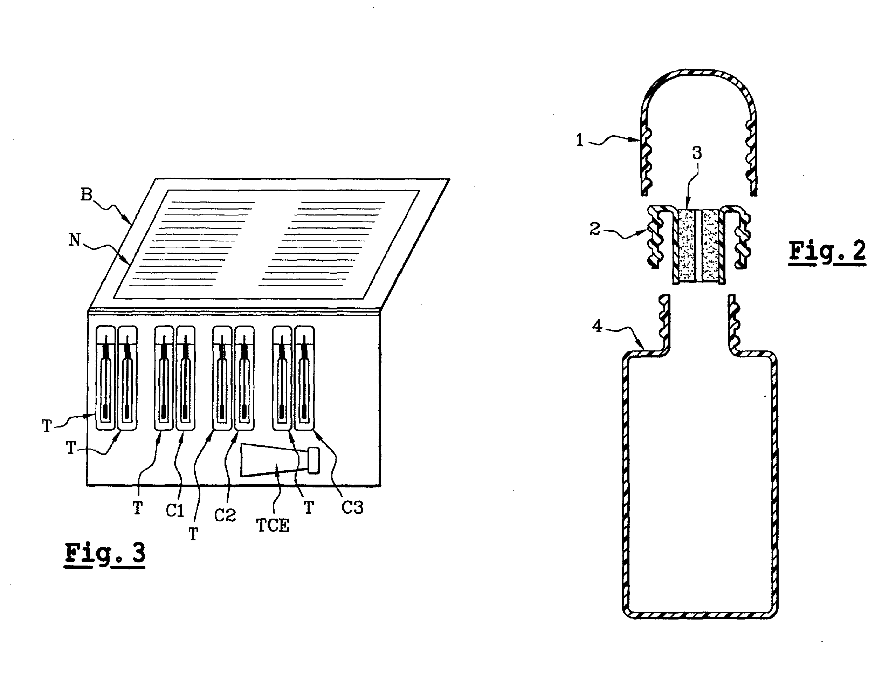

Ensemble (voir figure 1) qui permet de réaliser 5 kits multi usage pour environ 250 utilisations et comprend dans une boite B les éléments suivants :
1. Un portoir P permettant de faire tenir debout 7 flacons tels que décrits au point 4 de la présente liste;
2. Un flacon F1 contenant une solution hydroalcoolique à 10% (par exemple de 300 ml);
3. Un flacon F2 contenant une solution mère de capsaïcine à 1.10⁻²% (par exemple de 100 ml) dans une solution hydroalcoolique à 10%;
4. 40 flacons F3 vides bouchés (par exemple de 20 ml); Un flacon F3 (voir figure 2) est composé d'un récipient 4 adapté à recevoir un premier bouchon 2 qui permet d'éviter les coulures et d'essorer les cotons tige lors du prélèvement des solutions au moyen d'une partie centrale 3 en mousse découpée de manière à permettre le passage d'un coton tige, un bouchon standard 1 s'adaptant au premier bouchon;
5. 600 cotons tiges standards CT;
6. Une notice d'utilisation N contenant les éléments suivants :
   a) Un mode d'emploi pour la préparation et le flaconnage des solutions C1-C5 (3,16.10⁻ 5% (C1), 1.10⁻⁴% (C2), 3,16.10⁻⁴% (C3), 1.10⁻³% (C4) et 3,16.10⁻³% (C5)) et qui comprend la série d'étapes suivante :
      - Remplir un flacon F3T avec 15 ml de la solution hydroalcoolique à 10% de F1 ;
      - Remplir un flacon F3 avec 15 ml de solution du flacon F2; Nommer ce flacon F3C6;
      - Prélever 5 ml de ce flacon F3C6 et le transvaser dans un second flacon F3C5; Ajouter dans F3C5 10 ml de la solution hydroalcoolique de F1;
      - Reproduire itérativement les étapes précédentes pour obtenir F3C1-4;
   b) Un mode d'emploi pour la mise en oeuvre du test qui reprend les protocoles de l'exemple 1 en utilisant les cotons tiges CT et les flacons F3C1-6 et F3T préparés plus haut. Les flacons doivent être conservés à 4°C entre deux usages et jetés au bout d'une semaine d'utilisation;
   c) Un système d'étalonnage (atlas) permettent une interprétation des résultats (C1 : peau excessivement sensible ; C2: peau très sensible, C3: peau modérément sensible, C4 : peau assez peu sensible, C5 : peau peu sensible, C5 non détectée : peau non sensible).

### EXEMPLE 3 : Exemple de réalisation d'un kit visant à détecter le niveau de peau sensible, le kit étant destiné à un utilisateur professionnel en institut.

Ce kit qui permet de réaliser environ 50 utilisations et comprend dans une boite (B) les éléments suivants :
1. Un portoir P permettant de faire tenir debout 7 flacons tels que décrits au point 2 de la présente liste ;
2. 5 flacons F3 de 20ml contenant respectivement 15 ml des solutions C1-C5 (3,16.10⁻⁵% (C1), 1.10⁻⁴% (C2), 3,16.10⁻⁴% (C3), 1.10⁻³% (C4) et 3,16.10⁻³% (C5)) et un flacon F3T contenant 15 ml d'une solution hydroalcoolique à 10% T; Ces flacons sont représentés sur la figure 2 et correspondent à ceux qui ont été définis au point 4. de l'exemple 2;
3. 80 cotons tiges standards CT ;
4. Une notice d'utilisation contenant les éléments suivants :
   a) Un mode d'emploi pour la mise en oeuvre de test qui reprend le protocole de l'exemple 1 en utilisant les cotons tiges et les flacons F3C1-5 et F3T; Les flacons doivent être conservés à 4°C entre deux usages et jetés au bout d'une semaine d'utilisation.
   b) Un système d'étalonnage (atlas) permettent une interprétation des résultats (C1 : peau excessivement sensible C2: peau très sensible, C3: peau modérement sensible, C4 : peau assez peu sensible, C5 : peau peu sensible, C5 non détectée : peau non sensible).

### EXEMPLE 4 : Exemple de réalisation d'un kit visant à détecter le niveau de peau sensible, le kit étant destiné à un utilisateur professionnel ambulant.

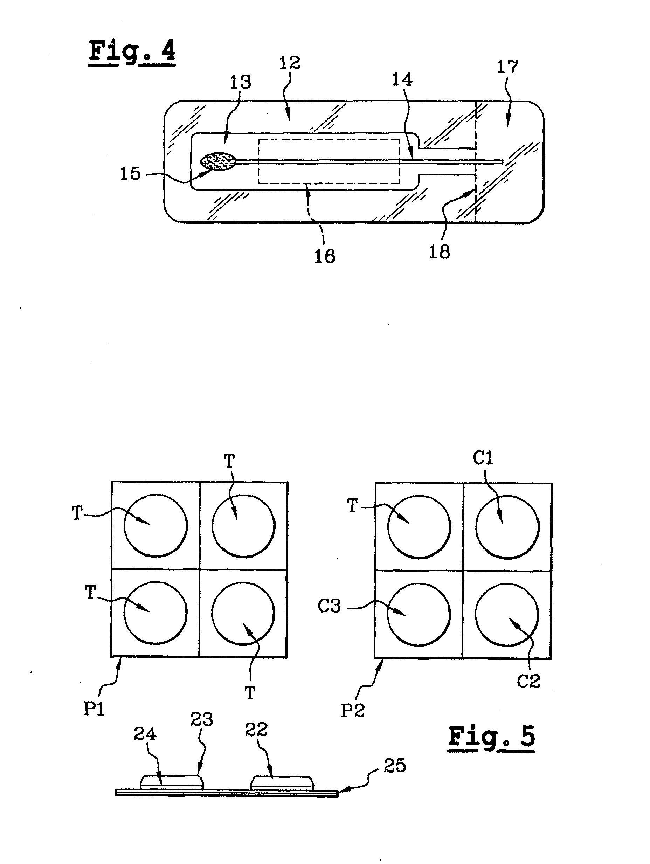

Ce kit mono-usage (voir figure 3) comprend dans une boîte B les éléments suivants :
1. 4 paires de monodoses M1-4 et M'1-4 contenant respectivement des solutions C1-C3 et des solutions T hydroalcooliques à 10% selon la présentation de la figure 2 et arrangées de la façon suivante: par paire T/T (étiquette rouge), T/C1 (étiquette orange), T/C2 (étiquette jaune) et T/C3 (étiquette verte). Une monodose (représentée à la figure 4) est une unité thermo-formée (blister) qui comprend une enveloppe externe 12 formée par deux films plastiques thermosoudés délimitant un compartiment central 13 qui contient une solution et un système d'application formé par une tige 14 et un embout 15. Une étiquette 16, qui peut être colorée, est collée sur la partie externe. Dans sa partie latérale 17, l'enveloppe externe enserre la partie distale de la tige dont elle reste solidaire après l'ouverture de la monodose par déchirement de l'enveloppe externe suivant la ligne de pointillé 18;
2. Un tube de crème émollient (TCE), apaisant;
3. Une notice d'utilisation N imprimée sur le couvercle contenant les éléments suivants :
   a) Un mode d'emploi pour la mise en oeuvre de test qui reprend les protocoles de l'exemple 1 en utilisant les monodoses M(')1-4. La crème émolliente peut être utilisée après le test en cas d'inconfort décrit par le consommateur. Le kit doit être jeté après usage.
   b) Un système d'étalonnage (atlas) permettent une interprétation des résultats (C1 : peau excessivement sensible C2: peau très sensible, C3: peau modérément sensible; peau peu ou pas sensible si C3 n'a pas été détectée).

### EXEMPLE 5 : Exemple de réalisation d'un kit visant à détecter le niveau de peau sensible, le kit étant destiné à un utilisateur privé.

Ce kit mono-usage est identique dans sa logique de fonctionnement à celui de l'exemple 4. La transposition à un usage privé est réalisée par une présentation qui assure une sécurité renforcée.

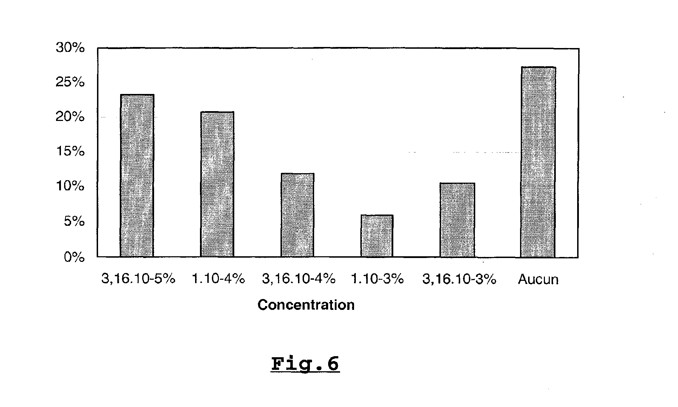

Le kit se présente sous la forme de deux «plaquettes» flexibles schématisées sur la figure 5. La première plaquette P1 contient quatre solutions témoins T, la seconde plaquette P2 contient quatre solutions T/C1/C2/C3. Chacune de ces solutions 22 est contenue dans une alvéole 23 et un gel microporeux 24 les sépare de la surface. Un film papier protecteur 24 assure l'intégrité lors du transport.

Par ailleurs, un système d'étalonnage (atlas) permettent une interprétation des résultats ainsi que l'identification des solutions de capsaïcine par rapport au véhicule est fournie dans une enveloppe fermée EF.

Les deux plaquettes P1 et P2 sont collées de façon symétrique sur les deux angles contralatéraux du maxillaire inférieur, après que le film protecteur ait été enlevé.

La procédure de test est la même que celle décrite précédemment, à ceci près que la mise en contact avec la peau est assurée par une pression des doigts sur les deux alvéoles symétriques de part et d'autre du visage, qui imbibe le gel microporeux.

Si aucune dose n'est détectée par une sensation dysesthésique, le sujet est déclaré à peau peu ou pas sensible. Si une dose est détectée, l'enveloppe fermée EF est ouverte et si le côté correspond à une concentration C1-3, le sujet est déclaré respectivement à peau excessivement sensible, Peau très sensible, ou Peau moyennement sensible.

**EXEMPLE 6 :** Exemple d'information quant à la neurosensibilité cutanée de l'individu en fonction de la concentration (X) de la première composition qui est détectée par l'individu par une sensation dysesthésique qui peut être donnée par le tableau de correspondance suivant:

| | |
|---|---|
| 1.10⁻⁶ % < X < 1.10⁻⁴% | neurosensibilité très élevée |
| 1.5 10⁻⁴ % < X < 0.75 10⁻³% | neurosensibilité moyennement élevée |
| 1. 10⁻³ % < X < 5. 10⁻³% | neurosensibilité faible |
| X > 1. 10⁻² % | neurosensibilité très faible |

### EXEMPLE 7: Utilisation du kit de l'exemple 3 pour la détermination du seuil de neurosensibilité cutanée de 150 femmes

Etude réalisée en simple aveugle sur une population tout venant de 150 femmes âgées de 18 à 60 ans et habitant Paris ou sa région.

### Résultats:

La figure 6 (Fig. 6) est un histogramme qui représente l'incidence des seuils de détection le seuil de détection de
Ce graphique montre qu'il existe plusieurs niveaux de neurosensibilité significativement distincts dans une population tout venante de 150 femmes âgées de 18 à 60 ans. Rappelons que les concentrations de capsaïcine sont espacées d'un facteur 3.16.
Ce graphique fait ressortir deux populations différentes : les sujets ayant détecté C1, C2 ou C3 (56%) forment une population « sensible à très sensible », les sujets ayant détecté C4, C5, ou n'ayant détecté aucune concentration (44%) forment une population « peu ou pas sensible ».
Il est intéressant de noter que les sensations rapportées au niveau du seuil de détection (caractérisé par une sensation détectée du côté d'application de la capsaïcine et durant au moins 30 secondes) sont très variées (présence, fourmillement, picotement, échauffement ....).

La cinétique de la sensation a été détaillée sur 74 des 150 sujets :

| **N** | **N manquant** | **Moyenne** | **Ecart-type** | **Minimum** | **Maximum** |
|---|---|---|---|---|---|
| 74 | 76 | 79.9 | 16.7 | 60 | 120 |

Globalement et en moyenne sur 74 sujets, le délai d'apparition est de 79.9 secondes soit 1 minute et 20 secondes

### Bonne Tolérance du test

Un point essentiel était d'évaluer si, à la concentration détectée pour l'arrêt du test, le sujet pouvait ressentir un inconfort significatif.
Il a donc été demandé aux 109 femmes ayant détecté une concentration, d'évaluer, sur une échelle de 1 à 5, l'intensité de cette sensation. Seules des sensations de niveau 2 (« léger mais perceptible » ; 95%) et 3 (« modéré clairement perceptible » ; 5%) ont été rapportées. Aucune sensation de niveau 4 (« importante ») ou 5 (« douloureux ») n'a été rapportée.
Cette étude démontre que la concentration d'attaque, ainsi que les paliers sont judicieusement choisis pour cette population parisienne.

### EXEMPLE 9: Procédé non thérapeutique d'identification des personnes à peaux sensibles par application topique cutanée d'une concentration unique de capsaïcine (solution hydroéthanolique de capsaïcine)

### Design expérimental

1) appliquer 3 fois de suite sur le sillon naso-génien, une solution hydroéthanolique à 10% d'éthanol de capsaïcine à une concentration de 5.10⁻⁴% ;
2) noter durant toutes les minutes et durant 5 minutes si la personne a ressentie une sensation dysesthésique, sur le sillon naso-génien ayant reçue la solution;
3) affecter un score de la sensation dysesthésique ressentie sur une échelle de 0 à 3 (0: absence de sensation; 1: sensation très légère; 2: sensation légère; 3: sensation modérée);
4) la personne est déclarée à peau sensible si le score est supérieur ou égal à 5 après 5 minutes ou si une sensation modérée est ressentie au cours des trois premières minutes.

**EXEMPLE 10 :** Procédé non thérapeutique d'identification des personnes à peaux sensibles par application topique cutanée d'une solution d'acide lactique à 10% puis d'une concentration unique de capsaïcine (solution hydroéthanolique de capsaïcine)

### Design expérimental

a) appliquer 10 fois de suite sur le sillon naso-génien, une solution d'acide lactique à une concentration de 10%;
b) noter durant toutes les minutes et durant 5 minutes si la personne a ressentie une sensation dysesthésique, sur la zone cutanée ayant reçue la solution;
c) affecter un score pour la sensation dysesthésique ressentie sur une échelle de 0 à 3 (0: absence de sensation; 1: sensation très légère; 2: sensation légère; 3: sensation modérée) ;
d) Passer à l'étape suivante si le score et supérieur ou égal à 5 après 5 minutes ou si une sensation légère ou modérée ou sévère est ressentie au cours des trois premières 3 minutes ;
e) appliquer 3 fois de suite sur le sillon naso-génien, une solution hydroéthanolique à 10% d'éthanol de capsaïcine à une concentration de 1.10⁻⁴%
f) noter durant toutes les minutes et durant 5 minutes si la personne a ressentie une sensation dysesthésique, sur la zone cutanée ayant reçue la solution;
g) affecter un score pour chaque sensation dysesthésique ressentie sur une échelle de 0 à 3 (0: absence de sensation; 1: sensation très légère; 2: sensation légère; 3: sensation modérée) ;
h) la personne est déclarée à peau sensible si, le score et supérieur ou égal à 5 après 5 minutes ou si au moins une sensation modérée est ressentie au cours des trois premières minutes.

## Revendications

1. Procédé non thérapeutique d'évaluation du niveau de neurosensibilité cutanée d'un individu consistant à :
1) appliquer sur une zone cutanée dudit individu une composition contenant un véhicule physiologiquement acceptable et un agent stimulant du système nerveux périphérique, la concentration dudit agent étant comprise entre 1.10⁶ et 5.10⁻⁴ % en poids par rapport au poids total de la composition;
2) noter si l'individu détecte ou ressent une sensation dysesthésique et en déduire une information quant à la neurosensibilité cutanée de l'individu, avantageusement de la réactivité ou la sensibilité cutanée.

2. Procédé non thérapeutique d'évaluation du niveau de neurosensibilité cutanée d'un individu suivant la revendication 1 consistant à :
1) appliquer sur une zone cutanée dudit individu une première composition contenant un véhicule physiologiquement acceptable et un agent stimulant du système nerveux périphérique, la concentration dudit agent étant comprise entre 1.10⁻⁶ et 1.10⁻⁴% en poids par rapport au poids total de la composition;
2a) noter si l'individu détecte une sensation dysesthésique;
2b) si aucune sensation n'est détectée par l'individu, recommencer les étapes 1) et 2a) avec une composition contenant une concentration plus élevée du même agent jusqu'à ce que l'individu détecte une sensation dysesthésique ou jusqu'à ce qu'à ce qu'une composition contenant une valeur maximale de concentration dudit agent soit appliquée;
2c) déduire, de la dernière concentration appliquée, une information quant à la neurosensibilité cutanée de l'individu.

3. Procédé suivant la revendication 2, **caractérisée en ce qu'**à l'étape 2b), la concentration de l'agent dans la composition est telle que l'application de la dite composition n'est pas susceptible d'engendrer des sensations dysesthésiques douloureuses chez l'individu.

4. Procédé suivant l'une quelconque des revendications 2 à 3, **caractérisée en ce qu'**à l'étape 2b) la concentration de l'agent augmente d'un facteur compris entre 1,5 à 10, de préférence entre 2 et 5.

5. Procédé suivant l'une quelconque des revendications 2 à 4, **caractérisée en ce que** la concentration augmente d'un facteur de racine carrée de 10.

6. Procédé suivant l'une quelconque des revendications 2 à 5 **caractérisée en ce que** la concentration de l'agent dans la première composition appliquée à l'étape 1) est comprise entre 3.10⁻⁶ et 6.10⁻⁵%.

7. Procédé suivant l'une quelconque des revendications 2 à 6 **caractérisée en ce que** la concentration de l'agent dans la première composition appliquée à l'étape 1) estde 3.16 10⁻⁵ %.

8. Procédé suivant l'une quelconque des revendications 2 à 7, **caractérisée en ce que** le véhicule physiologiquement acceptable est choisi parmi les solutions aqueuses, hydroalcooliques ou huileuses; les dispersions du type lotion ou sérum; les gels anhydres ou lipophiles; les émulsions de consistance liquide ou semi-liquide du type lait, obtenues par dispersion d'une phase grasse dans une phase aqueuse (H/E) ou inversement (E/H); les suspensions ou les émulsions de consistance molle, semi-solides ou solides, les microémulsions, les microcapsules, les microparticules; les dispersions vésiculaires de type ionique et/ou non ionique, avantageusement, les solutions, de préférence hydroalcooliques avec une teneur en alcool inférieure à 50%.

9. Procédé suivant l'une quelconque des revendications 2 à 8, **caractérisée en ce que** l'agent stimulant du système nerveux périphérique est un agent qui induit une réponse sensorielle liée à la mise en jeu de nerfs sensitifs cutanés.

10. Procédé suivant l'une quelconque des revendications 2 à 9, **caractérisée en ce que** l'agent stimulant du système nerveux périphérique est une substance capable d'induire une sensation dysesthésique lorsqu'il est appliqué topiquement sur la peau et d'induire une libération de substance P et/ou de CGRP lorsqu'il est appliqué topiquement sur la peau.

11. Procédé suivant l'une quelconque des revendications 2 à 10, **caractérisée en ce que** l'agent stimulant du système nerveux périphérique est choisi parmi les capsaicinoids naturels ou synthétiques, de préférence la capsaïcine, l'homocapsaïcine, homodihydrocapsaïcine, la nordihydrocapsaïcine, la dihydrocapsaïcine, avantageusement la capsaïcine; l'acide lactique, l'acide glycolique, l'éthanol à une concentration supérieure à 50%, l'huile de moutarde.

12. Procédé suivant l'une quelconque des revendications 2 à 11, **caractérisée en ce que** la zone cutanée est le pli du bras, le lobe de l'oreille ou le visage, en particulier l'aile du nez, le sillon naso-génien ou l'angle du maxillaire inférieur, de préférence l'aile du nez.

13. Procédé suivant l'une quelconque des revendications 2 à 12, **caractérisée en ce que** lorsque l'agent stimulant du système nerveux périphérique est la capsaïcine, le véhicule préféré est une solution hydroalcoolique, avantageusement hydroéthanolique.

14. Procédé suivant la revendication précédente, **caractérisée en ce que** la solution hydroéthanolique comprend de 1% à 50% d'éthanol dans l'eau, avantageusement de 5% à 20%, préférentiellement de 8% à 15% et plus préférentiellement 10%.

15. Procédé suivant l'une quelconque des revendications 2 à 14, **caractérisé en ce que** la sensation dysesthésique est la plus petite sensation non douloureuse ressentie dans une zone traitée par l'agent stimulant évoquant des picotements, des fourmillements, des démangeaisons ou prurits, des échauffements, des tiraillements, et/ou toute autre gêne ou inconfort telle qu'une sensation d'occlusion et/ou de vibrations.

16. Procédé suivant l'une quelconque des revendications 2 à 15, **caractérisé en ce que** l'étape 1) est précédée par l'application préalable sur une zone cutanée d'une composition comprenant le véhicule sans agent stimulant.

17. Procédé suivant la revendication précédente comprenant les étapes successives suivantes:
a1) appliquer sur une zone cutanée, une composition contenant le véhicule;
b1) noter si le sujet a ressenti une sensation dysesthésique sur la zone cutanée ayant reçue le véhicule ;
c1) si oui, arrêter le test; si non, appliquer sur une zone cutanée, de préférence sur la même zone ayant reçue précédemment le véhicule, le même véhicule contenant ledit agent stimulant à une concentration comprise entre 1.10⁶ et 1.10⁻⁴ ;
d1) noter si le sujet a ressenti une sensation dysesthésique, sur la zone cutanée ayant reçue la composition contenant l'agent stimulant;
e1) si oui, noter la concentration en agent stimulant et arrêter le test, si non, incrémenter la concentration en agent stimulant d'un facteur compris entre 1,5 et 10, et répéter n fois les étapes c1) à e1), avec n compris entre 1 et 10.

18. Procédé suivant la revendication précédente, **caractérisée en ce qu'**après l'étape a1) et avant l'étape b1) et/ou après l'étape c1) et avant l'étape d1), on attend 30 à 360 secondes, préférentiellement 120 à 200 secondes, particulièrement 180 secondes.

19. Procédé suivant l'une quelconque des revendications 2 à 14, **caractérisé en ce que** l'étape 1) est précédée par l'application préalable sur une zone cutanée sur sa zone contralatérale d'une composition comprenant le véhicule sans agent stimulant.

20. Procédé suivant la revendication précédente, comprenant les étapes successives suivantes:
a2) appliquer sur une zone cutanée et sur sa zone contralatérale, une composition contenant le véhicule;
b2) noter si le sujet a ressenti une sensation dysesthésique, sur l'une au moins des zones du visage ayant reçue le véhicule,
c2) si oui arrêter le test ; si non, appliquer sur une zone cutanée, le même véhicule contenant ledit agent stimulant à une concentration d'attaque comprise entre 1.10⁻⁶ et 1.10⁻⁴ %; et appliquer le même véhicule sur la zone contralatérale;
d2) noter si le sujet a ressenti une sensation dysesthésique discriminante, sur la zone cutanée ayant reçue le véhicule contenant l'agent stimulant par rapport à la zone cutanée contralatérale ;
e2) si oui, noter la concentration en agent stimulant et arrêter le test, si non, incrémenter la concentration en agent stimulant d'un facteur compris entre 1,5 à 10, et répéter n fois les étapes 3) à 5), avec n compris entre 1 à 10.

21. Procédé suivant la revendication précédente, **caractérisée en ce qu'**après l'étape a2) et avant l'étape b2) et/ou après l'étape c2) et avant l'étape d2), on attend 30 à 360 secondes, préférentiellement 120 à 200 secondes, particulièrement 180 secondes.

22. Procédé non thérapeutique d'identification des personnes à peaux sensibles suivant la revendication 1 consistant à :
1) appliquer sur une zone cutanée d'un individu, une solution aqueuse ou hydroalcoolique, avantageusement hydroéthanolique, d'un agent choisi parmi les capsaicinoides ou l'huile de moutarde à une concentration comprise entre 1.10⁻⁶ et 5.10⁻⁴% et ;
2) déduire une information quant à la réactivité ou la sensibilité cutanée dudit individu en fonction de l'intensité des sensations dysesthésiques susceptibles d'être ressenties par le dit individu.

23. Procédé non thérapeutique d'identification des personnes à peaux sensibles suivant la revendication précédente, **caractérisée en ce que** l'étape 1) est précédée d'une étape 0) consistant à :
0) appliquer sur une zone cutanée d'un individu, une solution d'acide lactique à une concentration comprise entre 2 et 10% en poids par rapport au poids total de la composition, avantageusement à 10%.

24. Procédé suivant l'une quelconque des revendications 22 et 23, **caractérisée en ce que** la concentration de l'agent est comprise entre 5.10⁻⁵ et 5.10⁻⁴%; de préférence la concentration est de 1.10⁻⁴% en poids par rapport au poids total de la composition.

25. Procédé suivant l'une quelconque des revendications 22 à 24, **caractérisée en ce que** l'étape 1) comprend entre 1 et 3 application(s) de la solution, de préférence 3 applications.

26. Procédé suivant l'une quelconque des revendications 23 à 25, **caractérisée en ce que** l'étape 0) comprend entre 1 et 10 application(s) de solution d'acide lactique, avantageusement 10 applications.

27. Procédé suivant l'une quelconque des revendications 22 à 26, **caractérisée en ce que** la zone cutanée, est choisie parmi le pli du bras, le lobe de l'oreille ou la face postérieure du pavillon de l'oreille ou le visage, de préférence l'aile du nez ou le sillon naso-génien.

28. Procédé suivant l'une quelconque des revendications 22 à 27, **caractérisée en ce que** la solution hydroéthanolique d'un agent contient de 1 à 50% d'éthanol dans l'eau, avantageusement de 5% à 20%, de préférence 10%.

29. Procédé suivant l'une quelconque des revendications 22 à 28, **caractérisée en ce que** les capsaicinoides sont d'origine naturels ou synthétiques, des extraits synthétiques ou végétaux.

30. Procédé suivant l'une quelconque des revendications 22 à 29, **caractérisée en ce que** les capsaicinoides sont choisis parmi la capsaïcine, l'homocapsaïcine, homodihydrocapsaïcine, la nordihydrocapsaïcine, la dihydrocapsaïcine, avantageusement la capsaïcine.

31. Procédé suivant l'une quelconque des revendications 22 à 30, **caractérisée en ce que** les sensations dysesthésiques sont choisies parmi les picotements, les fourmillements, les démangeaisons ou prurits, les échauffements, les tiraillements, et/ou les inconforts.

32. Procédé de traitement cosmétique de la peau d'un individu qui comprend un procédé tels que décrit dans l'une quelconque des revendications précédentes et une étape consistant à traiter cosmétiquement la peau avec un produit cosmétique en fonction de la neurosensibilité cutanée qui a été évaluée.

33. Procédé non thérapeutique pour déterminer l'efficacité d'un traitement cosmétique apte à agir sur la neurosensibilité cutanée d'un individu qui comprend un procédé tels que décrit dans l'une quelconque des revendications 1 à 31, à traiter cosmétiquement la peau avec un produit cosmétique en fonction de la neurosensibilité cutanée qui a été évaluée, renouveler sur la peau traitée un procédé tel que décrit dans l'une quelconque des revendications 1 à 31, puis déduire de la comparaison des résultats avant et après traitement une indication relative à l'efficacité du traitement cosmétique.

34. kit comprenant:
- une pluralité de récipients qui contiennent chacun des concentrations croissantes d'agent stimulant du système nerveux périphérique en association avec un véhicule,
- au moins un récipient qui contient le véhicule seul et,
- des systèmes mono-applicateurs, préférentiellement des cotons-tige,
**caractérisé en ce qu'**au moins un récipient contient une concentration en agent stimulant du système nerveux périphérique comprise entre 1.10⁻⁶ et 1.10⁻⁴ %, avantageusement entre 3.10⁻⁶ et 6.10⁻⁵ %; préférentiellement 3.16 10⁻⁵ % en poids par rapport au poids total de la composition.

35. Utilisation d'un kit suivant la revendication précédente dans un procédé tel que décrit dans l'une quelconque des revendications 1 à 21.
